# EUROPEAN PATENT APPLICATION

(11) **EP 1 760 149 A1**
(43) Date of publication of application: **07.03.2007**
(21) Application number: 05737205.4
(22) Date of filing: 06.05.2005
(51) Int. Cl.: C12N 15/00, C12N 9/00, C12P 21/02, G01N 33/15, G01N 33/50, C07K 1/00

(54) **METHOD OF CONSTRUCTING MODIFIED PROTEIN**

(30) Priority: 07.05.2004 JP 2004139147
(71) Applicant: PRECISION SYSTEM SCIENCE CO., LTD., Matsudo-shi, Chiba, 271-0064 (JP)
(72) Inventor: TAJIMA, Hideji, PRECISION SYSTEM SCIENCE CO., LTD, Matsudo-shi, Chiba 2710064 (JP); TAKAHASHI, Masaaki PRECISION SYSTEM SCIENCE CO LTD, Matsudo-shi, Chiba 2710064 (JP); MIYASHITA, Yukiko, PRECISION SYSTEM SCIENCE CO LTD, Matsudo-shi, Saitama 2710064 (JP)
(74) Representative: Bohnenberger, Johannes
(86) International application number: PCT/JP2005/008362
(87) International publication number: WO 2005/108567

(57) **Abstract**

DNA derived from a microorganism is prepared from a sample containing the microorganism, degenerate primers are prepared based on an amino acid sequence conserved among known proteins nos. 1 through N all having the same type of function, PCR is performed using the degenerate primers in the presence of the DNA derived from the microorganism, the nucleotide sequence of DNA fragment amplified by the PCR is determined and the amino acid sequence coded for by the DNA fragment is also determined, homology between the amino acid sequence coded for by the DNA fragment and amino acid sequences of known proteins is evaluated, a known protein is discovered which has an amino acid sequence having homology with the amino acid sequence coded for by the DNA fragment, the region of the DNA coding for the discovered known protein which codes for the amino acid sequence having the homology is replaced by the DNA fragment to produce a modified DNA, and the modified DNA is expressed to produce a modified protein, thereby achieving high-throughput and efficient functional analysis and screening of a modified protein.

SEQUENCE LISTING

<110> Precision System Science Co., Ltd.

<120> Method for producing modified protein
<130> 05737205.4
<160> 6

<210> 1
<211> 23
<212> DNA
<213> Artificial Sequence

<220>
<223> sequence of PCR primer

<400> 1
gaycchaacy tscaraayat hcc                                                23

<210> 2
<211> 21
<212> DNA
<213> Artificial Sequence

<220>
<223> sequence of PCR primer

<400> 2
kassakytcr tcgtgnacyt g                                                  21

<210> 3
<211> 20
<212> DNA
<213> Artificial Sequence

<220>
<223> sequence of PCR primer

<400> 3
ggagctgctt agcctgcccg                                                    20

<210> 4
<211> 19
<212> DNA
<213> Artificial Sequence

<220>
<223> sequence of PCR primer

<400> 4
ggagatctgg ccccaaaag                                                     19

<210> 5
<211> 42
<212> DNA
<213> Artificial Sequence

<220>
<223> sequence of PCR primer

<400> 5
gcaggctaag cagcagtccg atcccaacct ccagaacatc cc                           42

<210> 6

<211> 24
<212> DNA
<213> Artificial Sequence

<220>
<223> sequence of PCR primer

<400> 6
gaacaagatc tcgtcgtgga cctg                                              24

## Description

### TECHNICAL FIELD

The present invention relates to a method for producing a modified protein, a method for producing a modified protein library, and a method for screening a modified protein. The present invention also relates to a method for inferring a function of protein derived from a microorganism.

### BACKGROUND ART

Methods of obtaining proteins having excellent functions include (1) a method of identifying a novel protein and analyzing its function and (2) a method of introducing a random mutation into an amino acid sequence of a known protein to produce a modified protein, and analyzing the function of the modified protein (see for example Japanese Patent Application Laid-open No. 2003-304870.

In method (1) above, PCR is performed using primers designed based on an amino acid sequence or nucleotide sequence which is conserved among known proteins or genes, and the PCR-amplified fragment is used as a probe to obtain a gene coding for the novel protein. However, in order to evaluate the function of the novel protein it is necessary to determine the total nucleotide sequence of the gene coding therefor, and since this is unknown apart from the amino acid sequence or nucleotide sequence which is conserved among known proteins or genes, a great deal of time and effort is required to determine the total nucleotide sequence of the gene coding for the novel protein. Moreover, when identifying a protein or gene derived from a microorganism in method (1), in order to obtain genome DNA and mRNA from the microorganism it may be necessary to culture the microorganism in isolation, which is impracticable if the microorganism cannot be cultured or if the culture conditions are unknown.

In method (2), meanwhile, high throughput functional analysis of the modified protein is possible because the modified protein can be prepared easily and rapidly. However, in many cases introducing a random mutation into an amino acid sequence of a known protein actually detracts from its function.

### DISCLOSURE OF THE INVENTION

It is therefore an object of the present invention to provide a method for producing a modified protein, method for producing a modified protein library and method for screening a modified protein capable of providing high-throughput and efficient functional analysis and screening of modified proteins because modified proteins which are expected to have improved functions can be prepared easily and rapidly.

It is also an object of the present invention to provide a method for inferring the function of a protein derived from a microorganism which does not require that the entire sequence of DNA coding for the protein be identified or that the microorganism be isolation cultured in order to infer the function of a protein derived from the microorganism.

To achieve the aforementioned objects, the present invention provides the method for producing a modified protein, method for producing a modified protein library, method for screening a modified protein and method for inferring a function of protein derived from a microorganism of (1) to (7) below.
(1) A method for producing a modified protein, comprising the following steps (a) through (g):
   (a) a step of preparing DNA derived from a microorganism from a sample containing the microorganism;
   (b) a step of preparing degenerate primers based on an amino acid sequence conserved among known proteins nos. 1 through N all having the same type of function;
   (c) a step of performing PCR using the degenerate primers in the presence of the DNA derived from the microorganism;
   (d) a step of determining the nucleotide sequence of DNA fragment amplified by the PCR and determining the amino acid sequence coded for by the DNA fragment;
   (e) a step of evaluating homology between the amino acid sequence coded for by the DNA fragment and amino acid sequences of known proteins and discovering a known protein comprising an amino acid sequence having homology with the amino acid sequence coded for by the DNA fragment;
   (f) a step of producing a modified DNA by substituting the DNA fragment for the region coding for the amino acid sequence having the homology in DNA coding for the known protein discovered in the step (e); and
   (g) a step of causing expression of the modified DNA to thereby produce a modified protein.
(2) A method for producing a modified protein library, comprising the following steps (a) through (h):
   (a) a step of preparing DNA derived from a microorganism from a sample containing the microorganism;
   (b) a step of preparing degenerate primers based on an amino acid sequence conserved among known proteins nos. 1 through N all having the same type of function;
   (c) a step of performing PCR using the degenerate primers in the presence of the DNA derived from the microorganism;
   (d) a step of determining the nucleotide sequence of DNA fragment amplified by the PCR and determining the amino acid sequence coded for by the DNA fragment;
   (e) a step of evaluating homology between the amino acid sequence coded for by the DNA fragment and amino acid sequences of known proteins and discovering a known protein comprising an amino acid sequence having homology with the amino acid sequence coded for by the DNA fragment;
   (f) a step of producing a modified DNA by substituting the DNA fragment for the region coding for the amino acid sequence having the homology in DNA coding for the known protein discovered in the step (e); and
   (g) a step of causing expression of the modified DNA to thereby produce a modified protein; and
   (h) a step of performing the steps (a) through (g) with respect to two or more types of samples containing microorganisms.
(3) A method for screening a modified protein having a specific function, comprising the following steps (a) through (i):
   (a) a step of preparing DNA derived from a microorganism from a sample containing the microorganism;
   (b) a step of preparing degenerate primers based on an amino acid sequence conserved among known proteins nos. 1 through N all having the same type of function;
   (c) a step of performing PCR using the degenerate primers in the presence of the DNA derived from the microorganism;
   (d) a step of determining the nucleotide sequence of DNA fragment amplified by the PCR and determining the amino acid sequence coded for by the DNA fragment;
   (e) a step of evaluating homology between the amino acid sequence coded for by the DNA fragment and amino acid sequences of known proteins and discovering a known protein comprising an amino acid sequence having homology with the amino acid sequence coded for by the DNA fragment;
   (f) a step of producing a modified DNA by substituting the DNA fragment for the region coding for the amino acid sequence having the homology in DNA coding for the known protein discovered in the step (e); and
   (g) a step of causing expression of the modified DNA to thereby produce a modified protein;
   (h) a step of performing the steps (a) through (g) with respect to two or more types of samples containing microorganisms; and
   (i) a step of evaluating a function of the modified protein to thereby screen a modified protein having a specific function.
(4) A method for inferring a function of protein derived from a microorganism, comprising the following steps (a) through (g) and (j):
   (a) a step of preparing DNA derived from a microorganism from a sample containing the microorganism;
   (b) a step of preparing degenerate primers based on an amino acid sequence conserved among known proteins nos. 1 through N all having the same type of function;
   (c) a step of performing PCR using the degenerate primers in the presence of the DNA derived from the microorganism;
   (d) a step of determining the nucleotide sequence of DNA fragment amplified by the PCR and determining the amino acid sequence coded for by the DNA fragment;
   (e) a step of evaluating homology between the amino acid sequence coded for by the DNA fragment and amino acid sequences of known proteins and discovering a known protein comprising an amino acid sequence having homology with the amino acid sequence coded for by the DNA fragment;
   (f) a step of producing a modified DNA by substituting the DNA fragment for the region coding for the amino acid sequence having the homology in DNA coding for the known protein discovered in the step (e); and
   (g) a step of causing expression of the modified DNA to thereby produce a modified protein; and
   (j) a step of evaluating a function of the modified protein and then inferring a function of the protein derived from the microorganism based on the function of the modified protein.
(5) The method according to Claim 4, wherein the degenerate primers prepared in the step (b) are capable of amplifying a region coding for a functional domain of known proteins nos. 1 through N in DNA coding for the known proteins nos. 1 through N.
(6) The method according to Claim 4 or 5, wherein the known protein discovered in the step (e) has the same kind of function as the known proteins nos. 1 through N.
(7) The method according to any of Claims 4 through 6, wherein the microorganism is a microorganism which cannot be cultured or for which the culture conditions are unknown.

### BRIEF DESCRIPTION OF THE DRAWINGS

Figure 1 shows electrophoresis results for genome DNA extracted from soil of high-temperature environments.
Figure 2 shows alignment results for the amino acid sequences of family A-type DNA polymerase derived from multiple microorganisms (Bacillus subtilis, Bacillus caldotenax, Thermus aquaticus, Thermus thermophilus, Escherichia coli).
Figure 3 shows alignment results (continuation of Figure 2) for the amino acid sequences of family A-type DNA polymerase derived from multiple microorganisms (Bacillus subtilis, Bacillus caldotenax, Thermus aquaticus, Thermus thermophilus, Escherichia coli).
Figure 4 shows electrophoresis results for PCR amplified fragments obtained by PCR using degenerate primers in the presence of genome DNA extracted from multiple microorganisms (Bacillus caldotenax, Bacillus caldolyticus, Escherichia coli, Bacillus subtilis, Lactobacillus bulgaricus, Lactobacillus homohiochii, Lactobacillus heterohiochii, Thermus aquaticus, Thermus thermophilus, Sulfolobus solfataricus).
Figure 5 shows electrophoresis results for PCR amplified fragments obtained by PCR using degenerate primers in the presence of genome DNA collected from soil of high-temperature environments.
Figure 6 shows amino acid sequences coded for by PCR amplified fragments obtained by PCR using degenerate primers in the presence of genome DNA collected from soil of high-temperature environments.
Figure 7 shows electrophoresis results for a BlpI/BgIII fragment cut out from plasmid DNA.
Figure 8 shows the results of CBB (Coomassie brilliant blue) staining of a raw DNA polymerase fraction following SDS-PAGE.
Figure 9 shows electrophoresis results for a PCR amplified fragment obtained by PCR using modified DNA polymerase.
Figure 10 shows alignment results for the amino acid sequences of chitinase derived from multiple microorganisms (Serratia marcescens, Bacillus circulans, Stenotrophomonas maltophilia, Janthinobacterium lividum, Thermococcus kodakaraensis, Clostridium thermocellum, Bacillus licheniformis).
Figure 11 shows alignment results for the amino acid sequences of alanine dehydrogenase derived from multiple microorganisms (genera Bacillus, Carnobacterium, Synechocystis, Phormidium, Vibrio, Shewanella, Mycobacterium)
Figure 12 shows alignment results for the amino acid sequences of D-2-deoxyribose-5-phosphate aldolase derived from multiple microorganisms (Bacillus subtilis, Oceanobacillus iheyensis, Escherichia coli, Thermotoga maritima, Aquifex aeolicus).

### BEST MODE FOR CARRYING OUT THE INVENTION

The steps included in the methods of the present invention are explained in detail below.

### Step (a)

Step (a) is a step of preparing DNA derived from a microorganism from a sample containing the microorganism.

The type of sample is not particularly limited as long as it contains one or two or more types of microorganisms. Samples that can be used included for example soil, water, mud, sediment and other natural samples collected from various environments. The environment from which a natural sample is collected is not particularly limited, and natural samples may be collected from environments under various conditions (various temperature conditions such as low, normal and high, various pH conditions such as neutral, acidic and alkaline, various pressure conditions such as low, normal and high pressure). Specific examples of environments for collecting natural samples include hot springs and other high-temperature environments, acid hot springs and other acidic environments, alkali hot springs and other alkaline environments, and deep sea and other high-pressure environments.

The sample used is preferably one containing two or more types of microorganisms. Using a sample containing two or more types of microorganisms, the likelihood is higher of obtaining a PCR amplified fragment in step (c). There is also a higher likelihood of obtaining two or more types of PCR amplified fragments, so that multiple kinds of modified proteins can be obtained from 1 sample. The two or more types of PCR amplified fragments are obtained as a mixture, but the type of each PCR amplified fragment can be distinguished in step (d) by determining the nucleotide sequence of each PCR amplified fragment.

There are no particular limits on the type of microorganism contained in the sample, and examples include bacteria, molds, Basidiomycetes, yeasts, viruses and the like.

When a natural sample obtained from a high-temperature environment is used, the sample probably contains thermophiles. "Thermophile" is a general term for bacteria capable of living at high temperatures, normally indicating bacteria capable of living at 55°C or more. Thermophiles include extreme thermophiles capable of ordinarily living at 75°C or more, hyperthermophiles capable of ordinarily living at 85 to 90°C or more, facultative thermophiles capable of also living at normal temperature (normally 37°C), obligatory thermophiles capable of living only at about 40°C or more and the like.

The types (family, genus, species, etc.) of microorganisms contained in a sample may be identified or not identified. When the type of a microorganism contained in a sample is not identified, it is not necessary to identify the type of that microorganism.

A microorganism contained in a sample may be a microorganism that cannot be cultures or one for which the culture conditions are unknown. In the present invention there is no need to isolation culture a microorganism contained in a sample, which is particularly useful when a microorganism contained in a sample cannot be cultured or when its culture conditions are unknown.

DNA derived from a microorganism may be genome DNA or cDNA. Genome DNA from a microorganism can be extracted from the microorganism by ordinary methods. cDNA from a microorganism can be prepared by reverse transcribing mRNA extracted from the microorganism by ordinary methods. Genome DNA or mRNA ordinarily includes all the genome DNA or mRNA of the microorganism, but may include only part of the genome DNA or mRNA of the microorganism.

### Step (b)

Step (b) is a step of preparing degenerate primers based on an amino acid sequence conserved among known proteins nos. 1 through N all having the same kind of function.

Known proteins nos. 1 through N are natural proteins with known amino acid sequences, derivations, functions and the like.

There is no particular limit on the same kind of function which known protein nos. 1 through N have, but examples include heat resistance, DNA polymerase activity, chitinase activity, alanine dehydrogenase activity, D-2-deoxyribose-5-phosphate aldolase activity, nuclease activity, helicase activity and the like. In functions of known proteins nos. 1 through N, one kind of function may be the same, and two or more kinds of functions are the same.

There is no particular limit on the organisms from which known proteins nos. 1 through N are derived, but preferably they derive from a microorganism which is a close relative of the microorganism contained in the sample. When the microorganism contained in the sample is a close relative of the organisms from which known proteins nos. 1 through N are derived, there is a higher likelihood of obtaining a PCR amplified fragment in step (c). Even if the microorganism contained in the sample is unidentified, known proteins nos. 1 through N can be selected as being proteins derived from organisms which are close relatives of the microorganism contained in the sample. For example, when using a natural sample collected from a high-temperature environment, the natural sample is assumed to contain thermophiles, and therefor proteins derived from thermophiles can be selected as known proteins nos. 1 through N.

Degenerate primers can be prepared based on an amino acid sequence conserved among known proteins nos. 1 through N. That is, the amino acid sequences of known proteins nos. 1 through N are aligned, a conserved sequence (homologous sequence) is identified, the nucleotide sequences of the degenerate primers are determined based on the amino acid sequence of the conserved region, and the degenerate primers can then be prepared by an ordinary method such as chemical synthesis.

The degenerate primers include primers capable of hybridizing with the sense chain and primers capable of hybridizing with the antisense chain of double-stranded DNA coding for known proteins nos. 1 through N, and each of these primers can be prepared based on the amino acid sequence of a different conserved region.

It is thought that of DNA derived from the microorganism, that part of the DNA coding for a protein having the same type of function as known proteins nos. 1 through N is amplified by PCR using degenerate primers prepared based on the amino acid sequence conserved among known proteins nos. 1 through N, in the presence of the DNA derived from the microorganism.

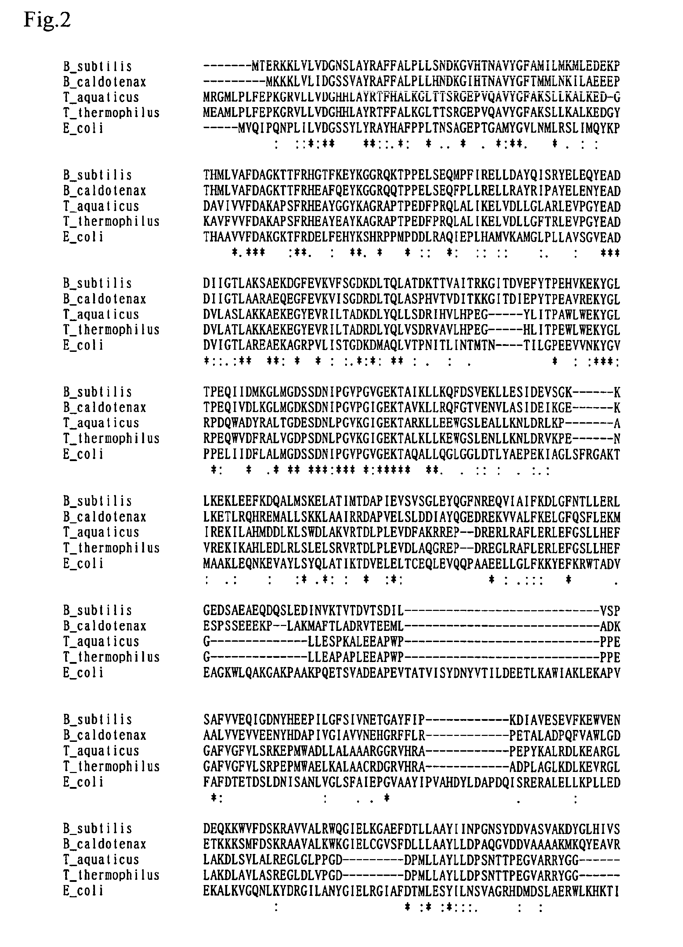

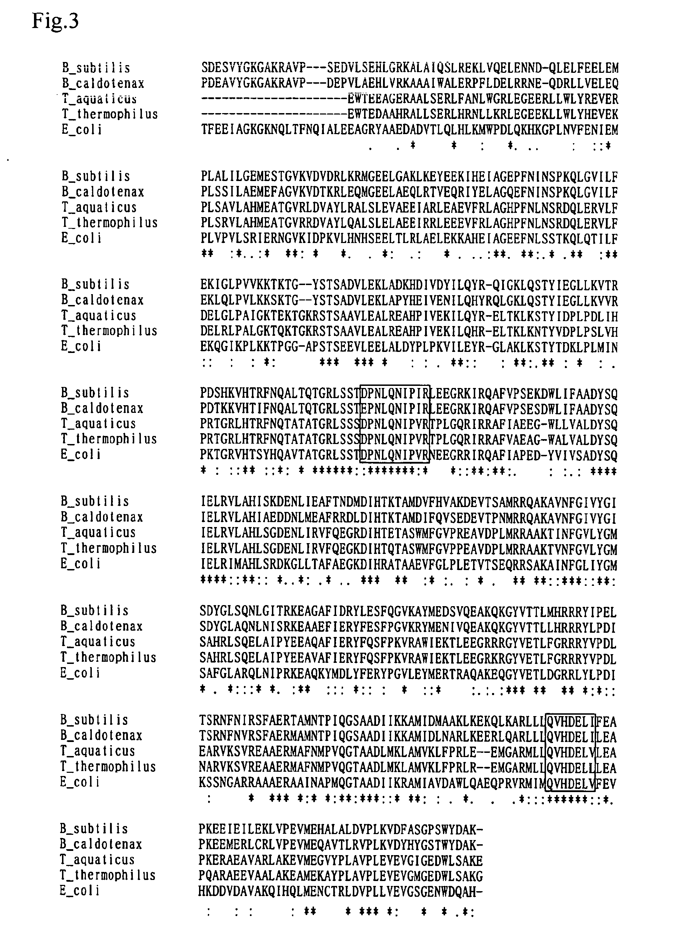

For example, as shown in Figures 2 and 3, amino acid sequences such as DPNLQNIP, QVHDELX (with X indicating I, V or L) and the like are conserved among family A-type DNA polymerase derived from microorganisms such as Bacillus subtilis, Bacillus caldotenax, Thermus aquaticus, Thermus thermophilus, Escherichia coli and the like, so the degenerate primer represented by 5'-gaycchaacytscaraayathcc-3' can be prepared based on DPNLQNIP and the degenerate primer represented by 5'-kassakytcrtcgtgnacytg-3' based on QVHDELX. A PCR amplified product can then be obtained by PCR using these degenerate primers in the presence of DNA derived from any microorganism (such as Bacillus caldotenax, Bacillus caldolyticus, Escherichia coli, Bacillus subtilis, Lactobacillus bulgaricus, Lactobacillus homohiochii, Lactobacillus heterohiochii, Thermus aquaticus, Themus thermophilus or Sulfolobus solfataricus). The resulting PCR amplified fragment is believed to be a DNA fragment amplified using as the template DNA coding for family A-type DNA polymerase (part of DNA coding for family A-type DNA polymerase).

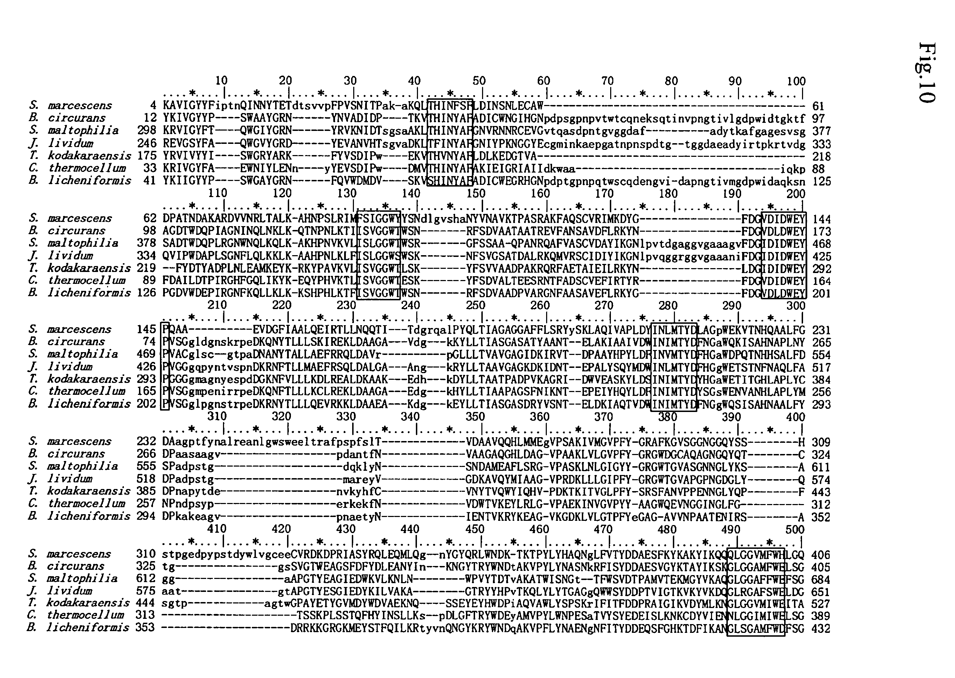

For example, as shown in Figure 10, amino acid sequences such as THINYAF, ISVGGWT, DIDWEYP, INVMTYD and GLGGAMFWE are conserved among kinase derived from microorganisms such as Serratia marcescens, Bacillus circulans, Stenotrophomonas maltophilia, Janthinobacterium lividum, Thermococcus kodakaraensis, Clostridium thermocellum and Bacillus licheniformis, and the degenerate primer represented by 5'-acncayathaaytaygcntt-3' can be prepared based on THINYAF, the degenerate primer 5'-athwsigtiggnggntggac-3' based on ISVGGWT, the degenerate primer 5'-gayathgaytgggartaycc-3' based on DIDWEYP, the degenerate primer 5'-athaaygtnatgacntayga-3' based on INVMTYD and the degenerate primer 5'-tcccadatcatiacnccncciarncc-3' based on GLGGAMFWE. A PCR amplified product can then be obtained by PCR using these degenerate primers in the presence of DNA derived from any microorganism. The resulting PCR amplified product is believed to be a DNA fragment (part of DNA coding for kinase) amplified with kinase-coding DNA as the template.

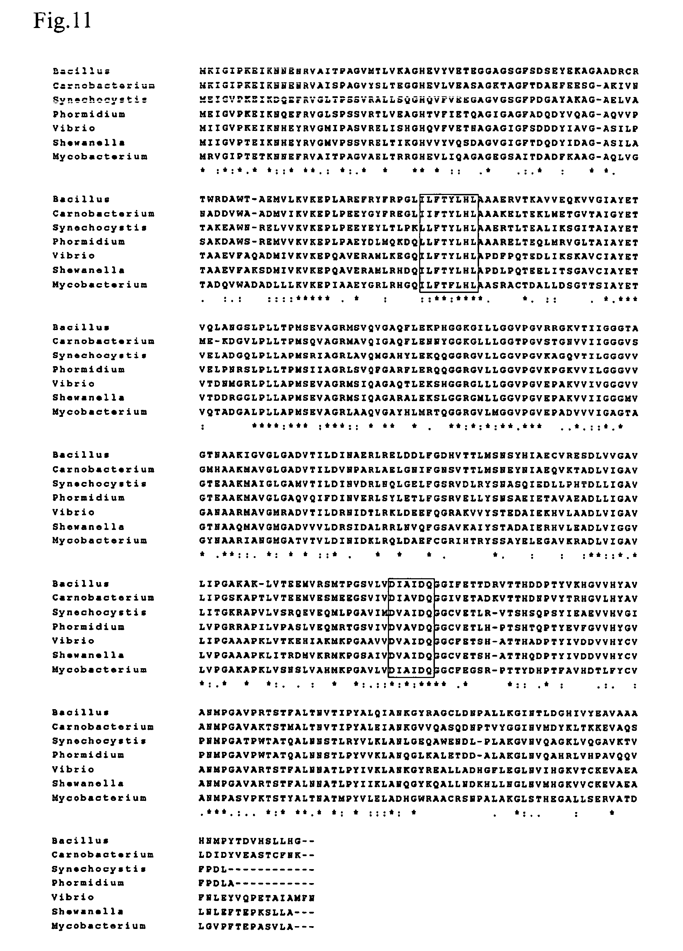

For example, as shown in Figure 11, amino acid sequences such as FTYLHLA and DVAIDQG are conserved among alanine dehydrogenase (AlaDH) derived from microorganisms in the genera Bacillus, Carnobacterium, Synechocystis, Phormidium, Vibrio, Shewanella, Mycobacterium and the like, and the degenerate primer represented by 5'-ttyacitwyyticayytigc-3' can be prepared based on FTYLHLA and the degenerate primer 5'-ccytgrtcdatigciayrtc-3' based on DVAIDQG. A PCR amplified product can then be obtained by PCR using these degenerate primers in the presence of DNA derived from any microorganism. The resulting PCR amplified product is believed to be a DNA fragment (part of DNA coding for alanine dehydrogenase) amplified with alanine dehydrogenase-coding DNA as the template.

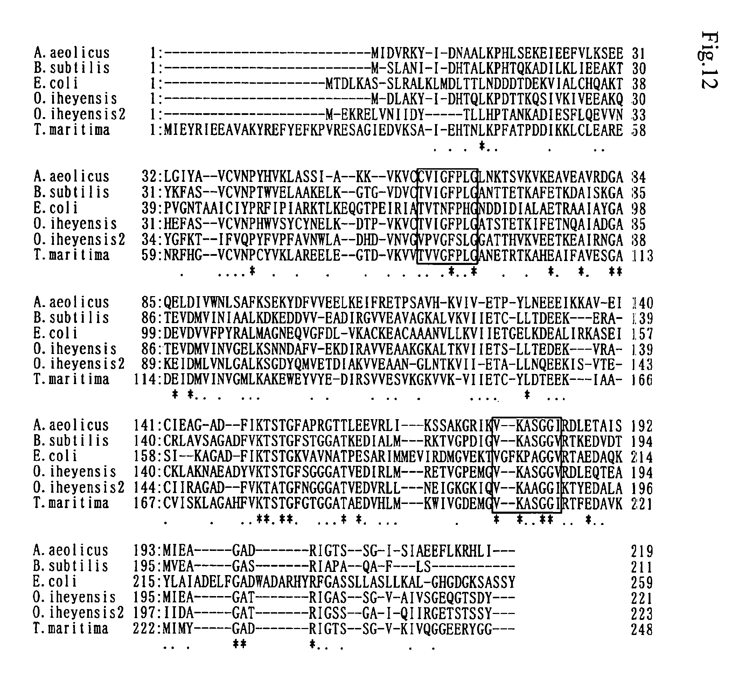

For example, as shown in Figure 12, amino acid sequences such as VIGFPLG and VKASGGV are conserved among D-2-deoxyribose-5-phosphate aldolase (DERA) derived from microorganisms such as Bacillus subtilis, Oceanobacillus iheyensis, Escherichia coli, Thermotoga maritima and Aquifex aeolicus, and the degenerate primer represented by 5'-gtnathggittycciytigg-3' can be prepared based on VIGFPLG and the degenerate primer represented by 5'-ayiccicciswngcyttnac-3' based on VKASGGV. A PCR amplified product can then be obtained by PCR using these degenerate primers in the presence of DNA derived from any microorganism. The resulting PCR amplified product is believed to be a DNA fragment (part of DNA coding for D-2-deoxyribose-5-phosphate aldolase) amplified with DNA coding for D-2-deoxyribose-5-phosphate aldolase as the template.

In the nucleotide sequences of the aforementioned degenerate primers, "i" represents inosine, "w" represents a or t, "y" represents t or c, "s" represents c or g, "k" represents g or t, "r" represents a or g, "h" represents a or t or c, "n" represents a or g or c or t, and "d" represents a or t or g.

A degenerate primer is an oligonucleotide consisting of normally 15 to 30 nucleotides or preferably 20 to 25 nucleotides, and can be chemically synthesized by ordinary methods. A label such as a restriction enzyme recognition sequence, tag sequence, fluorescent dye or radioisotope can be added to a degenerate primer.

The degenerate primers are preferably prepared so as to be capable of amplifying a region coding for a functional domain of known proteins nos. 1 through N in DNA coding for known proteins nos. 1 through N. A "functional domain" here is a domain involved in a function shared by known proteins nos. 1 through N (for example, a domain involved in DNA polymerase activity when the function shared by known proteins nos. 1 through N is DNA polymerase activity). It is thought that when a degenerate protein is capable of amplifying a region coding for a functional domain of known proteins nos. 1 through N, it can also amplify a region coding for a functional domain of a protein having the same kind of function as known proteins nos. 1 through N in DNA derived from the microorganism, so that when a modified protein is produced using such a PCR amplified fragment, not only is the modified protein more likely to have an enhanced function, but the function of the modified protein is more likely to reflect the function of a protein derived from a microorganism.

### Step (c)

Step (c) is a step of performing PCR using the degenerate primers in the presence of the DNA derived from the microorganism.

By performing PCR using degenerate primers in the presence of DNA derived from a microorganism it is possible to obtain a PCR amplified fragment with the DNA derived from the microorganism as the template. PCR can be performed by ordinary methods, and the PCR amplified fragment can be refined by an ordinary method such as polyacrylamide electrophoresis.

### Step (d)

Step (d) is a step of determining the nucleotide sequence of DNA fragment amplified by the PCR, and determining the amino acid sequence coded for by the DNA fragment.

The nucleotide sequence of the PCR amplified fragment can be determined by an ordinary method such as Maxam-Gilbert chemical modification, dideoxynucleotide chain termination or the like. A commercial nucleotide sequence analyzer for example can be used for analyzing the nucleotide sequence.

The PCR amplified fragment may be cloned by ordinary methods prior to nucleotide sequence analysis. For example, the PCR amplified fragment can be cloned by first incorporating it into a suitable cloning vector to prepare a recombinant vector which can then be used to transform E. coli or other host cells, after which the transformant is selected by means of a marker such as tetracycline resistance or ampicillin resistance. The cloning vector can be any capable of self-replication in host cells, such as a phage vector, plasmid vector or the like. E. coli or the like can be used for the host cells.

Since the determined nucleotide sequence may contain errors, it is preferably first corrected to remove errors before determining the amino acid sequence coded for by the nucleotide sequence.

### Step (e)

Step (e) is a step of evaluating homology between the amino acid sequence coded for by the DNA fragment and the amino acid sequences of known proteins, and discovering a known protein comprising an amino acid sequence having homology with the amino acid sequence coded for by the DNA fragment.

The known proteins to be evaluated for homology are natural proteins with known amino acid sequences, derivations, functions and the like, with no particular limitations as to type.

There are no particular limitations on the function of the known proteins to be evaluated for homology, which may be the same type of function as that of known proteins nos. 1 through N. or a different type of function from that of known proteins nos. 1 through N, but preferably it is the nos. 1 through N.

Homology can be evaluated using a known database or the like.

By evaluating homology it is possible to determine whether or not the amino acid sequence coded for by the PCR amplified fragment is a novel one. Steps (f) through (g) may be performed if the amino acid sequence coded for by the PCR amplified fragment is a known one, but are preferably performed if the amino acid sequence coded for by the PCR amplified fragment is a novel one.

By evaluating homology it is possible to determine whether a known protein has an amino acid sequence having homology with the amino acid sequence coded for by the PCR amplified fragment.

A region having homology is defined as a region having normally at least 50% or preferably at least 60% or more preferably at least 70% homology with the amino acid sequence coded for by the PCR amplified fragment.

### Step (f)

Step (f) is a step of producing a modified DNA by substituting the DNA fragment for the region coding for the amino acid sequence having the homology in DNA coding for the known protein discovered in step (e).

The modified DNA can be produced by treating DNA coding for the modified protein discovered in step (e) with restriction enzymes to remove the region coding for the amino acid sequence having homology with the amino acid sequence coded for by the PCR amplified fragment, and ligating the PCR amplified fragment to this part. The PCR amplified fragment is ligated so as to avoid a frame shift of the triplet (codon).

The modified DNA codes for a modified protein wherein the amino acid sequence having homology with the amino acid sequence coded for by the PCR amplified fragment has been replaced by the amino acid sequence coded for by the PCR amplified fragment in the amino acid sequence of the known protein discovered in step (e). Since the amino acid sequence coded for by the PCR amplified fragment is a naturally occurring amino acid sequence that has survived natural selection, it is assumed to have some kind of significance. Consequently, a modified protein wherein an amino acid sequence having homology with the amino acid sequence coded for by the PCR amplified fragment has been replaced by the amino acid sequence coded for by the PCR amplified fragment can be expected to have elevated function in comparison with a modified protein comprising a random mutation added to the amino acid sequence of a known protein.

The modified DNA comprises the open reading frame of the modified protein along with a termination codon at the 3' end. The modified DNA may also include an untranslated region (UTR) at the 5' end and/or 3' end of the open reading frame. Moreover, the modified DNA may also include open reading frames coding for other proteins or peptides so that the modified protein can be expressed as a fused protein.

### Step (g)

Step (g) is a step of causing expression of the modified DNA to produce a modified protein.

The modified protein can be produced for example as follows.

First, the modified DNA is inserted downstream from the promoter of a suitable expression vector to prepare a recombinant vector which is introduced into suitable host cells to prepare a transformant capable of producing the modified protein. Next, the transformant is cultured. When the modified protein is one that accumulates within the transformant cells, the culture is centrifuged to collect the cells in the culture, and the cells are then washed and crushed, and the modified protein extracted. When the modified protein is one that is excreted outside the transformant cells, the culture supernatant is either used as is or centrifuged to exclude the cells or bacterial bodies from the culture supernatant. The modified protein can be produced in this way.

The modified DNA needs to be incorporated into the recombinant vector in such a way that its function is exerted, and in addition to the promoter the recombinant vector may also include enhancers and other cis-elements, splicing signals, poly A addition signals, selection markers (such as a dihydrofolate reductase gene, ampicillin resistance gene or neomycin resistance gene), ribosome binding sequences (SD sequences) and the like.

The type of expression vector is not particularly limited as long as it is capable of self-replication in host cells, and examples include plasmid vectors, phage vectors, virus vectors and the like. Examples of plasmid vectors include E. coli-derived plasmids (for example, pRSET, pBR322, pBR325, pUC118, pUC119, pUC18 and pUC19), B. subtilis-derived plasmids (for example, pUB110 and pTP5) and yeast-derived plasmids (for example, Yep13, Yep24 and YCp50), while examples of phage vectors include λ-phages (such as Charon4A, Charon21A, EMBL3, EMBL4, λgt10, λgt11, λZAP) and examples of virus vectors include retroviruses, Vaccinia viruses and other animal viruses and Baculoviruses and other insect viruses.

The host cells are not particularly limited as to type as long as they can express the modified DNA, and examples include prokaryotic cells, yeasts, animal cells, insect cells, plant cells and the like.

The recombinant vector may be introduced for example by the electroporation method, spheroplast method, lithium acetate method, calcium phosphate method, lipofection method or the like.

The transformant may be cultured by an ordinary method used for culturing host cells.

The modified protein may be purified by solvent extraction, salting-out/desalination with ammonium sulfate or the like, precipitation with an organic solvent, dimethylaminoethyl (DEAE) Sepharose, ion-exchange chromatography, hydrophobic chromatography, gel filtration, affinity chromatography or the like.

### Step (h)

Step (h) is a step of performing steps (a) through (g) with respect to two or more types of samples containing microorganisms.

By performing steps (a) through (g) with respect to two or more types of samples, it is possible to produce a modified protein library that is an assemblage of two or more types of modified proteins.

Soil, water, mud, sediment or other natural samples collected from environments of differing temperature, pH values or the like can be used as the two or more types of samples.

### Step (i)

Step (i) is a step of evaluating a function of the modified protein to screen a modified protein having a specific function.

The function of the modified protein may be analyzed by ordinary methods. Functional analysis of the modified protein is performed at least with respect to the functions of the known protein before modification, but may also be performed with respect to other functions.

The specific function of the modified protein being screened is not particularly limited and may be selected appropriately according to the object.

### Step (j)

Step (j) is a step of evaluating a function of the modified protein and then inferring a function of the protein derived from the microorganism based on the function of the modified protein.

Since the modified protein has a part (amino acid sequence coding for PCR-amplified fragment) of the microorganism-derived protein, the function of the modified protein may reflect the function of the microorganism-derived protein. Since a functional domain is normally involved in a protein's function, when the part (amino acid sequence coding for PCR-amplified fragment) of the microorganism-derived protein in the modified protein is a functional domain of a protein having the same type of function as known proteins the 1st to N-th, the function of the modified protein is likely to reflect the function of the microorganism-derived protein. Moreover, since the part (amino acid sequence coding for PCR-amplified fragment) of the microorganism-derived protein in the modified protein is believed to be part of a protein having the same function as known proteins the 1st to N-th, when the known protein discovered in step (e) (protein into which part of microorganism-derived protein is incorporated) has the same kind of function as known proteins the 1st to N-th, it is highly likely that the function of the modified protein will reflect the function of the microorganism-derived protein.

### [Examples]

### [Example 1] Obtaining genome DNA from microorganisms contained in soil of high-temperature environments

### (1) Collecting high-temperature environment soil

It is thought that for DNA polymerase to be used effectively, it should have heat-resistance for use in PCR and the like. The most effective way of obtaining heat-resistant enzymes is from samples of high-temperature environments. High-temperature zones with differing properties (pH values) were therefore selected as high-temperature environments. One was the Kagoshima Kirishima Hot Spring region in Kyushu Prefecture, a strongly acidic environment, while the others were the Onikobe Hot Spring region in Miyagi Prefecture and the Daifunto Hot Spring region in Akita Prefecture, which have close to neutral pH.

Mud pots (holes filled with bubbling mud) exist in a natural state (untouched by humans) in specific areas within hot spring regions. Along with temperature, pH and other data, images and the like were recorded to show the color, moisture content and the like of samples collected from each of these locales. About 100 g of soil, mud or sediment was taken from each locale so that a sufficient quantity of DNA could be collected.

### (2) Extracting genome DNA from high-temperature environment soil

1 g of the collected soil was taken in a sterilized Eppendorf tube, and genome DNA derived from microorganisms contained in the soil was extracted. Genome DNA was extracted from the soil using an Ultra Clean^{™} Soil DNA Purification Kit (MO Bio, Catalog No. 12800-50, Funakoshi Catalog No. LM128000), a kit for DNA extraction from soil. Operations were carried out in accordance with the operations manual included with the kit.

2 µL or 1/50 of the actual extracted amount of DNA solution was taken and confirmed by 0.8% agarose gel electrophoresis after addition of 18 µL of TAE buffer (0.04 M Tris, 0.02 M acetic acid, 0.001 M EDTA (pH 8.0)) and 2µL of concentrated electrophoresis dye solution (0.25% bromophenol blue, 0.25% xylene cyanol, 30% glycerol).

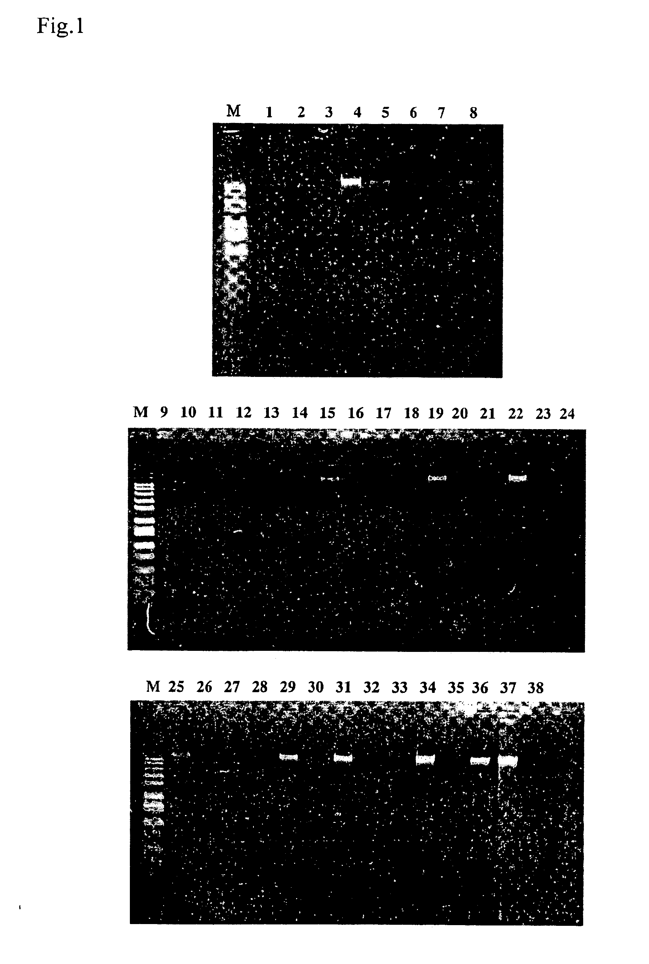

As a result, as shown in Figure 1, collection of genome DNA 10,000 nucleotide pairs or more in length was confirmed from various locales, although quantities differed. In Figure 1, "M" represents a molecular weight marker, and lanes 1 to 38 show results for different locales. These results confirm that microorganisms are present in high-temperature environment soil in a quantity that allows extraction of a sufficient amount of DNA.

### [Example 2] PCR using family A-type DNA polymerase-specific primers

### (1) Design of family A-type DNA polymerase-specific primers

Figures 2 and 3 show alignment results for the amino acid sequences of family A-type DNA polymerase derived from multiple microorganisms (Bacillus subtilis, Bacillus caldotenax, Thermus aquaticus, Thermus thermophilus, Escherichia coli). Figure 3 is a continuation of Figure 2.

In Figures 2 and 3, the two regions enclosed in rectangles (DPNLQNIP and QVHDELX (wherein X represents I, V or L)) are amino acid sequences sharing a high level of homology among the microorganisms. This amino acid sequence should be conserved in the family A-type DNA polymerase of other microorganisms.

The two degenerate primers (5' primer (SEQ ID NO:1) and 3' primer (SEQ ID NO:2)) shown in Table 1 were designed based on the aforementioned amino acid sequence (Takashi Uemori, Yoshizumi Ishino, Kayo Fujita, Kiyozo Asada and Ikunoshin Kato, "Cloning of the DNA polymerase gene of Bacillus caldotenax and characterization of the Gene Product" (1993), J. Biochem., 113, 401-410).

**[Table 1]**

| Type of primer | Nucleotide sequence |
|---|---|
| 5' primer | gaycchaacytscaraayathcc |
| 3' primer | kassakytcrtcgtgnacytg |

"Y" here represents t or c, "s" represents c or g, "k" represents g or t, "r" represents a or g, "h" represents a or t or c and "n" represents a or g or c or t.

### (2) PCR using family A-type DNA polymerase-specific primers

PCR was performed in a solution comprising 50 µL 10 mM Tris-HCl (pH 8.3), 50 mM KCl, 15 mM MgCl₂, 200 µM dNTP and 5 units of TAKARA ExTaq polymerase (Takara Bio) using 100 pmol of the aforementioned degenerate primers in the presence of 0.5 ng of genome DNA extracted from 10 species of microorganisms (Bacillus caldotenax, Bacillus caldolyticus, Escherichia coli, Bacillus subtilis, Lactobacillus bulgaricus, Lactobacillus homohiochii, Lactobacillus heterohiochii, Thermus aquaticus, Thermus thermophilus and Sulfolobus solfataricus). PCR was performed in 30 cycles of a temperature cycle consisting of 30 seconds at 94°C, 1 minute at 55°C and 2 minutes at 72°C.

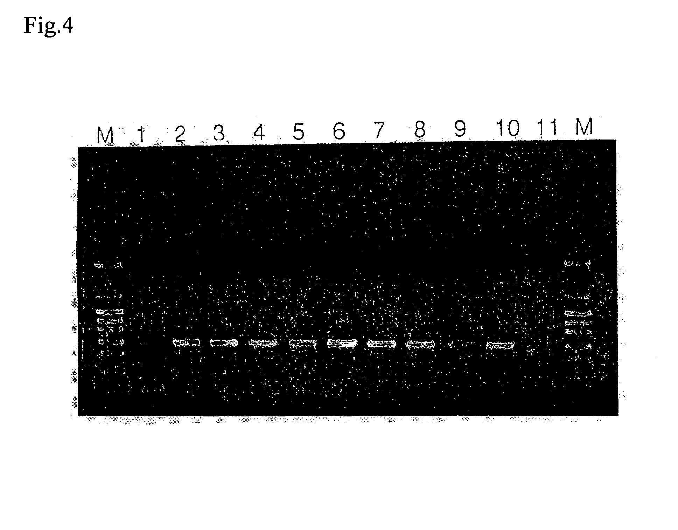

As a result, as shown in Figure. 4, a PCR amplified product presumed to derive from family A-type DNA polymerase was obtained from all 10 microorganisms. In Figure 4, "M" is a molecular weight marker, and Lane 2 shows results for Bacillus caldotenax, Lane 3 for Bacillus caldolyticus, Lane 4 for Escherichia coli, Lane 5 for Bacillus subtilis, Lane 6 for Lactobacillus bulgaricus, Lane 7 for Lactobacillus homohiochii, Lane 8 for Lactobacillus heterohiochii, Lane 9 for Thermus aquaticus, Lane 10 for Thermus thermophilus, and Lane 11 for Sulfolobus solfataricus.

These results show that these degenerate primers can be used as primers for family A-type DNA polymerase genes from any microorganism.

Next, PCR was performed in a solution comprising 50 µL 10 mM Tris-HCl (pH 8.3), 50 mM KCl, 15 mM MgCl₂, 200 µM dNTP and 5 units of TAKARA ExTaq polymerase (Takara Bio) using 100 pmol of the aforementioned degenerate primers in the presence of 1/50 (1 µL) the total collected amount of genome DNA collected from high-temperature environment soil of the Kirishima Hot Spring region, Onikobe Hot Spring region, etc. PCR was performed in 30 cycles of a cycle consisting of 30 seconds at 94°C, 1 minute at 55°C and 2 minutes at 72°C.

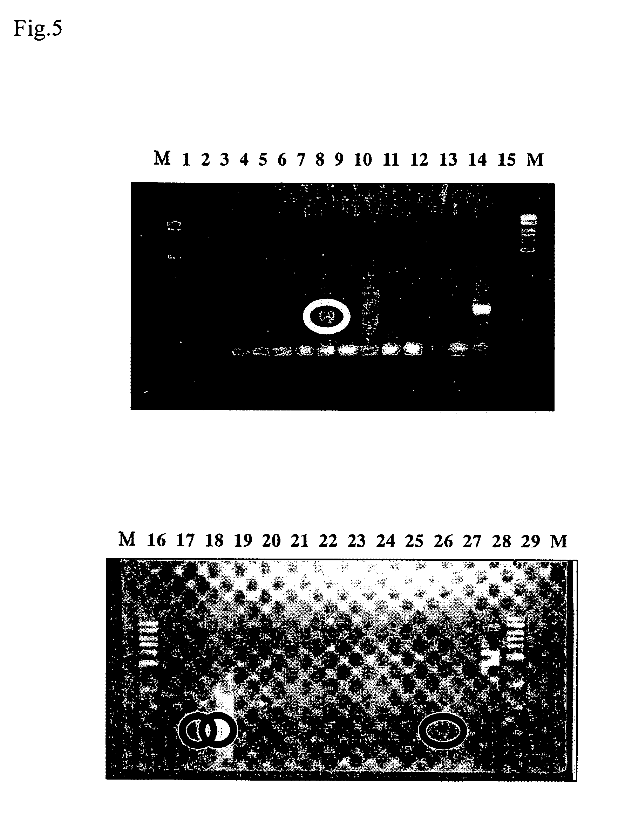

As a result, as shown in Figure 5, DNA fragments presumed to derive from family A-type DNA polymerase were amplified when genome DNA collected from certain locales was used as the template. When a DNA fragment was of roughly the expected length, it was presumed to derive from family A-type DNA polymerase. In Figure 5, "M" is a molecular weight marker, while Lanes 1 to 29 show results for various locales.

### (3) Cloning of amplified fragments

Equal amounts of 10 mM Tris-HCl (pH 7.5) and 1 mM EDTA solution were added to the reaction liquid after completion of PCR, water-saturated phenol/chloroform solution in an amount equal to that of the aqueous solution was added, thoroughly agitated and heated for 10 minutes at 70°C, followed by centrifugation at 10,000 rpm for 5 minutes, and the upper aqueous solution fraction was carefully isolated. The isolated aqueous solution fraction was added to a Microcon 100 (Millipore), and centrifuged for 15 minutes at 2,500 rpm. 200 µL of 10 mM Tris-HCl (pH 7.5) and 1 mM EDTA solution were again added and centrifuged at 2,500 rpm for 15 minutes. These operations served to remove the unreacted primers, nucleotides, salts, etc.

About 0.05 µg of the PCR amplified product was mixed with 0.1 µg of pGEM T-vector (Promega) dissolved in 10 mM Tris-HCl (pH 7.5) and 1 mM EDTA solution and with 8 µL of 10 mM Tris-HCl (pH 7.5)/1 mM EDTA solution, and 10 µL of Ligation Kit Version 2 (Takara Bio) was added and retained at 16°C for 30 minutes to ligate the PCR amplified fragment to the plasmid DNA. 1 µL of plasmid DNA with the PCR amplified fragment ligated thereto was added to 50 µL of calcium-treated E. coli DH10B, left standing for 20 minutes on ice and warmed for 2 minutes at 42°C, and 950 µL of liquid LB medium (containing 10 g tryptone, 5 g yeast extract, 5 g sodium chloride and 1 g glycose per 1 L) was then added to the cells, which were then shaking cultured for 60 minutes at 37°C. Upon completion of shaking culture, 150 µL of the culture was planted on LB agar medium containing 100 µg/mL of ampicillin, and cultured overnight at 37°C.

### (4) Decoding the nucleotide sequences of the PCR amplified fragments

100 µg/mL of the E. coli colony proliferated on the agar medium was planted in LB medium containing about 2 mL of ampicillin, and shaking cultured overnight at 37°C. The proliferated cells were collected by centrifugation for 10 minutes at 10,000 rpm, and plasmid DNA was collected using a QIAprep Spin Miniprep Kit (QIAGEN, Catalog No. 27104). PCR was performed with the collected plasmid DNA as the template using nucleotide sequence decoding primers (M13 primer M4 and T promoter primer). A dye terminator cycle sequencing kit (ABI) was used in the PCR, and the nucleotide sequence was decoded using an ABI 3700 automatic nucleotide sequence detector.

### (5) Determining amino acids sequence coded for by PCR amplified fragments

Since the determined nucleotide sequence of a PCR amplified fragment may include mechanical errors, the nucleotide sequences were modified by hand to remove mechanical errors, and the amino acid sequences coded for by the nucleotide sequence were determined. A homology search was performed using a public protein database, and the amino acid sequence codeds for by the PCR amplified fragments were compared with the amino acid sequence of a known family A-type DNA polymerase (DNA polymerase (TaqDNA polymerase) from Thermus aquaticus).

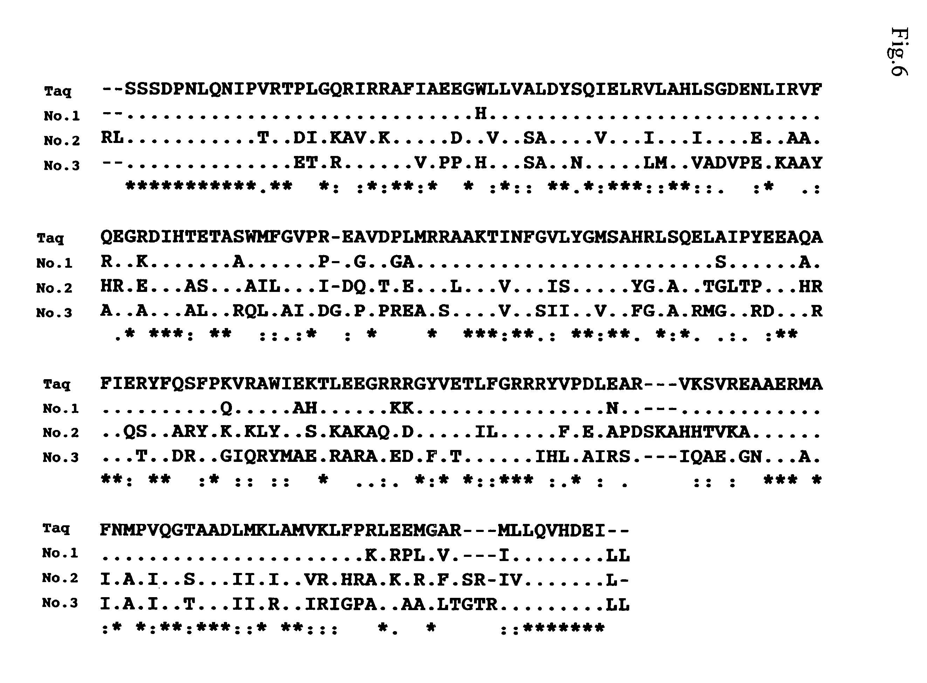

As a result, as shown in Figure 6, the amino acid sequences coded for by the PCR amplified fragments were confirmed to include the amino acid sequence of the functional domain of family A-type DNA polymerase.

In Figure 6, "Taq" represents the amino acid sequence of TaqDNA polymerase, and "No. 1", "No. 2" and No. 3" represent amino acid sequences coded for by PCR amplified fragments obtained using as the template genome DNA collected from different locales and having 90%, 57% and 52% homology, respectively, with the amino acid sequence of TaqDNA polymerase. The amino acid sequences represented by "No. 1", "No. 2" and "No. 3" are only the sequences that differ from TaqDNA polymerase.

Moreover, as shown in Table 2, the amino acid sequences coded for by PCR amplified fragments obtained using as templates genome DNA collected from different locales (a to g) each exhibited homology with the amino acid sequence of family A-type DNA polymerase derived from different microorganisms, confirming that many unknown family A-type DNA polymerases exist in high-temperature environments.

**[Table 2]**

| Sample No. | Microorganism | Homology |
|---|---|---|
| a | Anaerocellum thermophilum | 44% |
| b | Thermoanaerobacter yonseines | 52% |
| c | Thermomicrobium roseum | 55% |
| d | E. coli klenow fragment | 49% |
| e | Chlorobium tepidum | 61% |
| f | Thermus filiformis | 65% |
| g | Thermus aquaticus | 95% |

### [Example 3] Construction of plasmid having DNA coding for modified DNA polymerase

### (1) Design of primers for introducing restriction enzyme sites

Since the PCR amplified fragments obtained in Example 2 cover the region central to DNA polymerase activity, modified DNA polymerase in which that region of the amino acid sequence of TaqDNA polymerase having homology with the amino acid sequence coded for by a PCR amplified fragment is replaced by the amino acid sequence coded for by the PCR amplified fragment should have DNA polymerase activity different from that of TaqDNA polymerase, and could exhibit improved DNA polymerase activity. Moreover, the function of the modified DNA polymerase should reflect the function of an unknown family A-type DNA polymerase present in high-temperature environment soil.

Therefore, the two primers primer A (SEQ ID NO:3) and primer B (SEQ ID NO:4) shown in Table 3 were designed for purposes of introducing restriction enzyme sites (BlpI site and Bg1II site) into the outside of the region where a PCR amplified fragment was to be substituted in DNA coding for TaqDNA polymerase. Primers A and B were designed to introduce new restriction enzyme sites without being affected by the amino acid sequence of the TaqDNA polymerase.

Meanwhile, the two primers primer C (SEQ ID NO:5) and primer D (SEQ ID NO:6) shown in Table 3 were designed for purposes of introducing restriction enzyme sites (B1pI site and Bg1II site) into the outside of the PCR amplified fragment. Primers C and D were designed to introduce new restriction enzyme sites without being effected by the amino acid sequence coded for by the PCR amplified fragment.

**[Table 3]**

| Primer | Nucleotide sequence |
|---|---|
| A | ggagctgcttagcctgcccg |
| B | ggagatctggccccaaaag |
| C | gcaggctaagcagcagtccgatcccaacctccagaacatccc |
| D | gaacaagatctcgtcgtggacctg |

In Table 3, a single underline indicates a newly introduced B1pI site, while a double underline indicates a newly introduced Bg1II site.

### (2) PCR using primers for introducing restriction enzyme sites

PCR was performed in a solution containing 50 µL of 10 mM Tris-HCl (pH 8.3), 50 mM KCl, 15 mM MgCl₂, 200 µM of dNTP and 2.5 units of PfuUltra DNA polymerase (Stratagene) using 2 pmol of primers A and B in the presence of 10 ng of pTAQ9 vector containing DNA coding for TaqDNA polymerase (Ishino Y, Ueno T, Miyagi M, Uemori T, Imamura M, Tsunasawa S and Kato I, "Overproduction of Thermus aquaticus DNA polymerase and its structural analysis by ion-spray mass spectrometry" (1994), J. Biochem. (Tokyo), 116(5), 1019-24). PCR was carried out in 30 cycles of a thermal cycle consisting of 1 minute at 95°C, 1 minute at 55°C and 2 minutes at 72°C.

Next, PCR was performed in a solution containing 50 µL of 10 mM Tris-HCl (pH 8.3), 50 mM KCl, 15 mM MgCl₂, 200 µM of dNTP and 2.5 units of PfuUltra DNA polymerase (Stratagene) using 2 pmol of primers C and D in the presence of 10 ng of a plasmid clone obtained in Example 2. PCR was carried out in 30 cycles of a thermal cycle of 1 minute at 95°C, 1 minute at 55°C and 2 minutes at 72°C.

### (3) Restriction enzyme treatment of PCR amplified fragments

The PCR amplified fragments were cleaved with restriction enzymes B1pI and Bg1II. That is, 5 µL of 10 times concentrated restriction enzyme buffer (200 mM Tris-HCl (pH 8.2), 100 mM MgCl₂, 600 mM NaCl, 10 mM DTT), 42 µL of sterile distilled water and 3 µL of the restriction enzyme B1pI were added to 50 µL of each PCR amplification reaction liquid, thoroughly mixed, and retained at 37°C for 3 hours. Next, 4 µL of 5 M NaCl solution and 3µL of restriction enzyme Bg1II were added, thoroughly mixed, and retained at 60°C for 3 hours. Upon completion of warming, 10 µL of 10 mM EDTA and 2% SDS solution was added to complete the reaction.

Each cleaved DNA fragment was isolated by 0.8% agarose gel electrophoresis, and collected and purified using a QIAGEN QuiaQuickTip.

### (4) Ligation of restriction enzyme-treated DNA fragments

0.5 µg (1 µL) of each collected and purified DNA fragment was mixed on ice, 3 µL of 10 mM Tris-HCl (pH 7.5) and 1 mM EDTA solution was added, and 5 µL of Takara Bio Takara Ligation Kit Version 2 was then added, thoroughly mixed, and retained at 16°C for 1 hour.

### (5) Introduction into E. coli

1 µL of the ligation reaction liquid was mixed on ice with 20 µL of calcium-treated E. coli DH10B cell solution, left standing for 20 minutes, and heat treated for 2 minutes at 42°C. 980 µL of LB liquid medium was added, and the cells were shaking cultured for 50 minutes at 37°C. 100 µL was then taken and seeded uniformly on agar LB medium containing 100 µg/mL ampicillin, and retained overnight at 37°C.

### (6) Confirming recombination

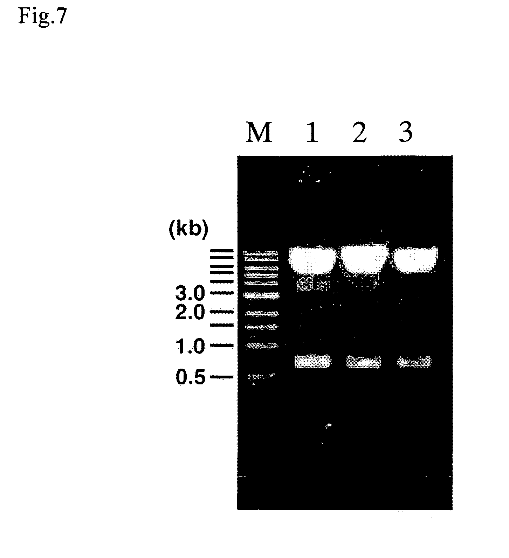

Of the E. coli cells seeded on the agar medium, only those having plasmid DNA proliferated and formed colonies on the agar medium. 10 of the resulting colonies were randomly selected, planted in 2 mL of LB liquid medium and shaking cultured overnight at 37°C, and plasmid DNA was collected using a QIAGEN QIAprep Spin Miniprep Kit (Catalog No. 27104). The collected plasmid DNA was treated with B1pI and Bg1II to cut off a B1pI/Bg1II fragment. As a result, incorporation of the PCR amplified fragment into a pTAQ9 vector containing DNA coding for TaqDNA polymerase or in other words construction of a pTAQ9 vector having DNA coding for modified DNA polymerase was confirmed as shown in Figure 7. In Figure 7, "M" represents a marker, and Lanes 1 to 3 show results for pTAQ9 vectors having incorporated PCR amplified fragments obtained using as templates genome DNA collected from differing locales.

### [Example 4] Expression and functional analysis of modified DNA polymerase

### (1) Introduction of vector into E. coli for expression

Two different pTAQ9 vectors each having DNA coding for different types of modified DNA polymerase were transformed into E. coli BL21-CodonPlus(DE3)-RIL (Stratagene), which has a high efficiency of expression of exogenous proteins.

Competent cells of E. coli BL21-CodonPlus(DE3)-RIL were thawed, and transferred 0.1 mL per tube into two Falcon tubes. Solution corresponding to 10 ng of two kinds of pTAQ9 vector was added separately to each, left standing for 30 minutes on ice and subjected to heat shock at 42°C for 30 seconds, after which 0.9 mL of SOC medium was added and the cells were shaking cultured for 1 hour at 37°C. A suitable quantity was then seeded on an LB agar plate containing ampicillin, and cultured overnight at 37°C to obtain transformed E. coli BL21-CodonPlus(DE3)-RIL/ST0452-1.

### (2) Expression of modified DNA polymerase

The transformant appearing on the agar medium was cultured overnight at 37°C in 5 mL of LB medium containing ampicillin, IPTG (Isopropyl-b-D-thiogalactopyranoside) was added to 1 mM, and the cells were cultured for 5 hours at 30°C. After culture, cells were collected by centrifugation (6,000 rpm, 20 minutes).

The collected cells were heat treated for 30 minutes at 75°C, and suspended by addition of 2 times the amount of 50 mM tris-hydrochloric acid buffer (pH 7.5), one tablet of protease inhibitor (Complete EDTA-free, Roche) and 0.5 mg of DNase RQ1 (Promega). The resulting suspension was ultrasound disrupted, retained at 37°C for 10 minutes, and centrifuged (11,000 rpm, 20 minutes) to obtain a supernatant. Ammonium sulfate was added to a final concentration of 0.2 M to this supernatant, which was then centrifuged for 10 minutes at 18,000 x g to obtain a supernatant. This supernatant was treated in a HiTrap Phenyl HP column and then in a HiTrap Heparin HP column to obtain a raw DNA polymerase fraction.

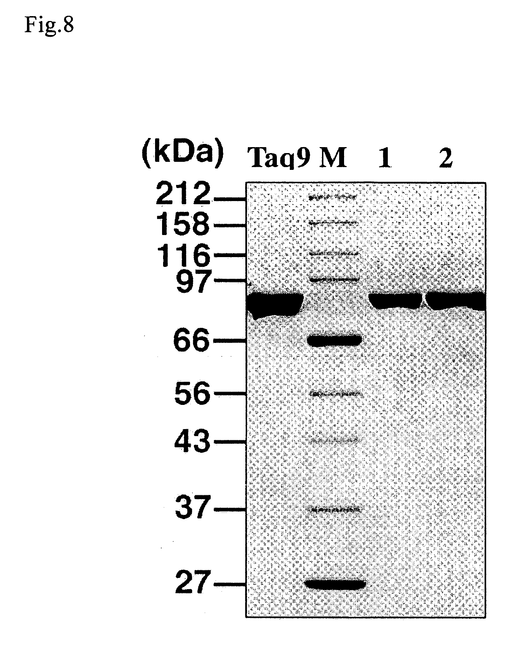

The raw DNA polymerase fraction was subjected to SDS-PAGE, and CBB (Coomassie brilliant blue) stained with the results shown in Figure 8. In Figure 8, "M" is a molecular weight marker, while "pTAQ9" indicates results for the raw DNA polymerase fraction obtained by expressing a pTAQ9 vector comprising DNA coding for TaqDNA polymerase, and Lanes 1 and 2 show results for raw DNA polymerase fractions obtained by expressing pTAQ9 vectors having DNA coding for modified DNA polymerase. As shown in Figure 8, the raw DNA polymerase fractions were roughly uniform protein samples.

### (3) Functional analysis of modified DNA polymerase

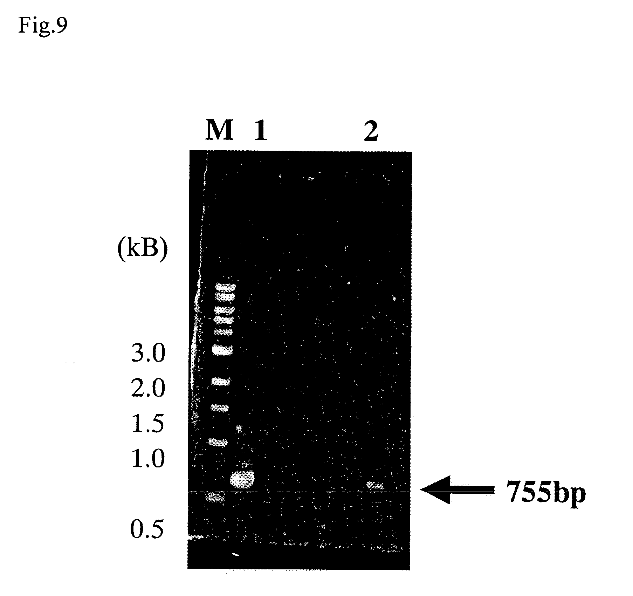

PCR was performed using the modified DNA polymerase to confirm whether or not it had heat-resistant DNA polymerase activity. 1 ng of pET32 plasmid DNA, 10 pmol each of T7 promoter primer and T7 terminator primer specific to that plasmid DNA (both from Novagen), and 1 µL of raw DNA polymerase liquid were mixed with 50 µL of buffer containing 10 mM Tris-HCl (pH 8.3), 50 mM KCl, 15 mM MgCl₂ and 200 µM of dNTP, and subjected to 30 cycles of a thermal cycle of 30 seconds at 94°C, 30 seconds at 55°C and 1 minute at 72°C. PCR was also performed under similar conditions using commercial TaqDNA polymerase (TaKaRa Taq) as a control. As a result, amplification of the predicted DNA region was confirmed as shown in Figure 9. These results show that the modified DNA polymerase has heat-resistant DNA polymerase activity. In Figure 9, "M" represents a molecular weight marker, while Lane 1 shows electrophoresis results for the PCR amplified fragment obtained using commercial TaqDNA polymerase (control), and Lane 2 shows electrophoresis results for the PCR amplified fragment obtained using modified DNA polymerase.

## Claims

1. A method for producing a modified protein, comprising the following steps (a) through (g):
(a) a step of preparing DNA derived from a microorganism from a sample containing the microorganism;
(b) a step of preparing degenerate primers based on an amino acid sequence conserved among known proteins nos. 1 through N all having the same type of function;
(c) a step of performing PCR using the degenerate primers in the presence of the DNA derived from the microorganism;
(d) a step of determining the nucleotide sequence of DNA fragment amplified by the PCR and determining the amino acid sequence coded for by the DNA fragment;
(e) a step of evaluating homology between the amino acid sequence coded for by the DNA fragment and amino acid sequences of known proteins and discovering a known protein comprising an amino acid sequence having homology with the amino acid sequence coded for by the DNA fragment;
(f) a step of producing a modified DNA by substituting the DNA fragment for the region coding for the amino acid sequence having the homology in DNA coding for the known protein discovered in the step (e); and
(g) a step of causing expression of the modified DNA to thereby produce a modified protein.

2. A method for producing a modified protein library, comprising the following steps (a) through (h):
(a) a step of preparing DNA derived from a microorganism from a sample containing the microorganism;
(b) a step of preparing degenerate primers based on an amino acid sequence conserved among known proteins nos. 1 through N all having the same type of function;
(c) a step of performing PCR using the degenerate primers in the presence of the DNA derived from the microorganism;
(d) a step of determining the nucleotide sequence of DNA fragment amplified by the PCR and determining the amino acid sequence coded for by the DNA fragment;
(e) a step of evaluating homology between the amino acid sequence coded for by the DNA fragment and amino acid sequences of known proteins and discovering a known protein comprising an amino acid sequence having homology with the amino acid sequence coded for by the DNA fragment;
(f) a step of producing a modified DNA by substituting the DNA fragment for the region coding for the amino acid sequence having the homology in DNA coding for the known protein discovered in the step (e); and
(g) a step of causing expression of the modified DNA to thereby produce a modified protein; and
(h) a step of performing the steps (a) through (g) with respect to two or more types of samples containing microorganisms.

3. A method for screening a modified protein having a specific function, comprising the following steps (a) through (i):
(a) a step of preparing DNA derived from a microorganism from a sample containing the microorganism;
(b) a step of preparing degenerate primers based on an amino acid sequence conserved among known proteins nos. 1 through N all having the same type of function;
(c) a step of performing PCR using the degenerate primers in the presence of the DNA derived from the microorganism;
(d) a step of determining the nucleotide sequence of DNA fragment amplified by the PCR and determining the amino acid sequence coded for by the DNA fragment;
(e) a step of evaluating homology between the amino acid sequence coded for by the DNA fragment and amino acid sequences of known proteins and discovering a known protein comprising an amino acid sequence having homology with the amino acid sequence coded for by the DNA fragment;
(f) a step of producing a modified DNA by substituting the DNA fragment for the region coding for the amino acid sequence having the homology in DNA coding for the known protein discovered in the step (e); and
(g) a step of causing expression of the modified DNA to thereby produce a modified protein;
(h) a step of performing the steps (a) through (g) with respect to two or more types of samples containing microorganisms; and
(i) a step of evaluating a function of the modified protein to thereby screen a modified protein having a specific function.

4. A method for inferring a function of protein derived from a microorganism, comprising the following steps (a) through (g) and (j):
(a) a step of preparing DNA derived from a microorganism from a sample containing the microorganism;
(b) a step of preparing degenerate primers based on an amino acid sequence conserved among known proteins nos. 1 through N all having the same type of function;
(c) a step of performing PCR using the degenerate primers in the presence of the DNA derived from the microorganism;
(d) a step of determining the nucleotide sequence of DNA fragment amplified by the PCR and determining the amino acid sequence coded for by the DNA fragment;
(e) a step of evaluating homology between the amino acid sequence coded for by the DNA fragment and amino acid sequences of known proteins and discovering a known protein comprising an amino acid sequence having homology with the amino acid sequence coded for by the DNA fragment;
(f) a step of producing a modified DNA by substituting the DNA fragment for the region coding for the amino acid sequence having the homology in DNA coding for the known protein discovered in the step (e); and
(g) a step of causing expression of the modified DNA to thereby produce a modified protein; and
(j) a step of evaluating a function of the modified protein and then inferring a function of the protein derived from the microorganism based on the function of the modified protein.

5. The method according to Claim 4, wherein the degenerate primers prepared in the step (b) are capable of amplifying a region coding for a functional domain of known proteins nos. 1 through N in DNA coding for the known proteins nos. 1 through N.

6. The method according to Claim 4 or 5, wherein the known protein discovered in the step (e) has the same kind of function as the known proteins nos. 1 through N.

7. The method according to any of Claims 4 through 6, wherein the microorganism is a microorganism which cannot be cultured or for which the culture conditions are unknown.
